# EUROPEAN PATENT APPLICATION

(11) **EP 4 813 305 A1**
(43) Date of publication of application: **30.09.2026**
(21) Application number: 25305433.2
(22) Date of filing: 25.03.2025
(51) Int. Cl.: A61B 6/00, A61B 6/50

(54) **PROVIDING VIEWING ANGLES FOR ACQUIRING X-RAY PROJECTION DATA**

(71) Applicant: Koninklijke Philips N.V., 5656 AE Eindhoven (NL)
(72) Inventor: GOOSSEN-NACHTIGALL, Peggy, 5656 Eindhoven (NL); OLIVAN BESCOS, Javier, 5656 Eindhoven (NL); ALLAIRE, Stéphane, 5656 Eindhoven (NL); HENDRIKS, Bernardus, 5656 Eindhoven (NL); BROSENS-KESSELS, Angelique, 5656 Eindhoven (NL); KONTAXIS, Charis, 5656 Eindhoven (NL); ELENBAAS, Thijs, 5656 Eindhoven (NL)
(74) Representative: Philips Intellectual Property & Standards

(57) **Abstract**

A system (100) for providing viewing angles for acquiring X-ray projection data representing a vascular region, is proposed. The system includes one or more processors (110) configured to: receive (S110) volumetric data (120) representing the vascular region (130). The processor(s) are also configured to determine (S120), based on the volumetric data (120), and for each of a plurality of portions (130_{1..i}) of the vascular region, a corresponding range of one or more viewing angles (Δα_{1..i}, Δβ_{1..i}) for acquiring X-ray projection data (150) representing the portion (130_{1..i}). The processor(s) are also configured to output (S130) the ranges for the portions (130_{1..i}) and/or an optimal range of one or more viewing angles (Δαₒₚₜ, Δβₒₚₜ) for acquiring X-ray projection data (150) representing the plurality of portions (130_{1..i}), the optimal range being determined based on the ranges for the portions (130_{1..i}).

## Description

### TECHNICAL FIELD

The present disclosure relates to providing viewing angles for acquiring X-ray projection data representing a vascular region. A system, a computer-implemented method, and a computer program product, are disclosed.

### BACKGROUND

Vascular procedures are often performed using projection X-ray images. For instance, digital subtraction angiography "DSA" images, are often generated in the context of assessing and treating a suspected brain aneurysm. The DSA images capture the aneurysm and its surrounding blood vessels, and are used to assess the aneurysm, and also to place treatment devices. If treatment is required, the images may be used to place metal coils in the vasculature in order to block-off the aneurysm, and thereby prevent it from rupturing. Similarly, projection X-ray images of the coronary vasculature are often generated in the context of a coronary interventional procedure in order to assess, and also treat, stenoses in coronary blood vessels.

However, a drawback of projection X-ray images is that they lack depth information. The lack of depth information in projection X-ray images can give a misleading impression of the vasculature, particularly if the images are acquired from a sub-optimal viewing angle with respect to the anatomy. They may give a misleading impression of the length, and also the diameter, of a stenosis in a blood vessel, for example. This can adversely affect both the assessment, and the treatment, of the vessel. This issue is particularly acute in the vascular procedures described above, not only because of the highly tortuous nature of the neural and coronary vasculatures, but also because of the extent of the vasculature that may be relevant to the procedure. For instance, in the case of a brain aneurysm, a physician is typically interested in both the location of a brain aneurism, and also its feeding vessel. There may also be multiple aneurysms that are distributed throughout the neural vasculature. Likewise, in a coronary interventional procedure there may be multiple, potentially extended, lesions, that require assessment and treatment. Such issues confound the generation of projection X-ray images that are easy to interpret.

Consequently, there is a need to improve the way in which viewing angles are determined for acquiring X-ray projection data that is used to generate projection X-ray images of the vasculature.

### SUMMARY

The invention is defined by the claims.

According to one aspect of the present disclosure, a system for providing viewing angles for acquiring X-ray projection data representing a vascular region, is provided. The system includes one or more processors configured to:
receive volumetric data representing the vascular region;
determine, based on the volumetric data, and for each of a plurality of portions of the vascular region, a corresponding range of one or more viewing angles for acquiring X-ray projection data representing the portion; and
output the ranges for the portions and/or an optimal range of one or more viewing angles for acquiring X-ray projection data representing the plurality of portions, the optimal range being determined based on the ranges for the portions.

Thus, the system provides ranges of viewing angles for acquiring X-ray projection data representing corresponding portions of the vascular region and/or an optimal range for acquiring X-ray projection data representing a plurality of portions of the vascular region. The optimal range is determined based on the ranges for the portions. The system may therefore be used to acquire X-ray projection data that facilitates an improved visualization of the vascular region.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing viewing angles for acquiring X-ray projection data representing a vascular region, in accordance with some aspects of the present disclosure.
Fig. 2 is a flowchart illustrating an example of a computer-implemented method of providing viewing angles for acquiring X-ray projection data representing a vascular region, in accordance with some aspects of the present disclosure.
Fig. 3 is a schematic diagram illustrating an example of the generation of a virtual projection 130^{VP} of a vascular region 130 from a viewing angle α₁, β₁, of a virtual projection X-ray imaging system 210^{V} with respect to a vascular region 130, in accordance with some aspects of the present disclosure.
Fig. 4 is a schematic diagram illustrating a first example of a viewing angle α of a projection X-ray imaging system 210 with respect to a vascular region 130, in accordance with some aspects of the present disclosure.
Fig. 5 is a schematic diagram illustrating a second example of a viewing angle β of a projection X-ray imaging system 210 with respect to a vascular region 130, in accordance with some aspects of the present disclosure.
Fig. 6 is a schematic diagram illustrating examples of ranges of viewing angles Δα₁ and Δα₂ for acquiring X-ray projection data representing individual portions 130₁ and 130₂ of a vascular region, and also an optimal range of viewing angles Δαₒₚₜ for acquiring X-ray projection data representing both portions 130₁, and 130₂, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, in a corresponding manner.

In the following description, reference is made to examples of a system for providing viewing angles for acquiring X-ray projection data representing portions of a vascular region. In some examples, the portions of the vascular region are located in the neural vasculature. It is, however, to be appreciated that the neural vasculature serves only as an example of part of the anatomy in which the portions of the vascular region may be located. In general, the portions of the vascular region may be located in any part of the anatomy. For instance, the portions of the vascular region may alternatively be located in the cardiac vasculature, or in the pulmonary vasculature, or in the peripheral vasculature, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a there is a need to improve the way in which viewing angles are determined for acquiring X-ray projection data that is used to generate projection X-ray images of the vasculature.

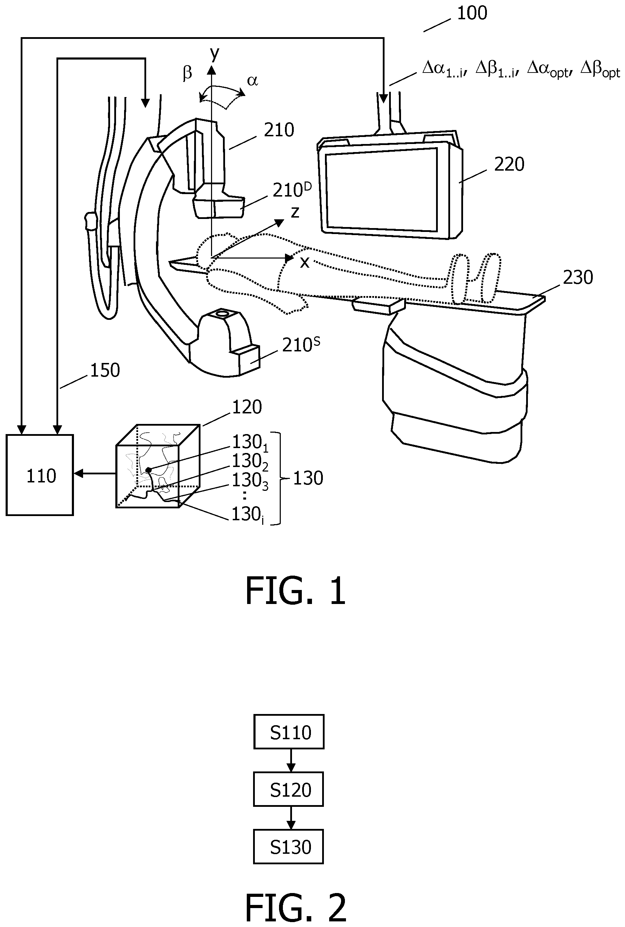

Fig. 1 is a schematic diagram illustrating an example of a system 100 for providing viewing angles for acquiring X-ray projection data representing a vascular region, in accordance with some aspects of the present disclosure. Fig. 2 is a flowchart illustrating an example of a computer-implemented method of providing viewing angles for acquiring X-ray projection data representing a vascular region, in accordance with some aspects of the present disclosure. It is noted that operations that are described as being performed by the processor(s) 110 of the system 100 illustrated in Fig. 1, may also be performed in the method illustrated in Fig. 2. Likewise, operations that are described in relation to the method described with reference to Fig. 2 may also be performed by the processor(s) 110 of the system 100 illustrated in Fig. 1. With reference to Fig. 1, and Fig. 2, the system 100 for providing viewing angles for acquiring X-ray projection data representing a vascular region includes one or more processors 110 configured to:
receive S110 volumetric data 120 representing the vascular region 130;
determine S120, based on the volumetric data 120, and for each of a plurality of portions 130_{1..i} of the vascular region, a corresponding range of one or more viewing angles Δα_{1..i}, Δβ_{1..i} for acquiring X-ray projection data 150 representing the portion 130_{1..i}; and
output S130 the ranges for the portions 130_{1..i} and/or an optimal range of one or more viewing angles Δαₒₚₜ, Δβₒₚₜ for acquiring X-ray projection data 150 representing the plurality of portions 130_{1..i}, the optimal range being determined based on the ranges for the portions 130_{1..i}.

Thus, the system provides ranges of viewing angles for acquiring X-ray projection data representing corresponding portions of the vascular region and/or an optimal range for acquiring X-ray projection data representing a plurality of portions of the vascular region. The optimal range is determined based on the ranges for the portions. The system may therefore be used to acquire X-ray projection data that facilitates an improved visualization of the vascular region.

The operations that are performed by the processor(s) 110 of the system 100 are described in more detail below.

Referring initially to the operation S110 illustrated in Fig. 2; in this operation, the processor(s) 110 receive volumetric data 120. The volumetric data 120 represents a vascular region 130.

The volumetric data 120 may represent a vascular region that is located in various parts of the anatomy. For instance, the vascular region may be located in the neural vasculature, or in the cardiac vasculature, or in the pulmonary vasculature, or in the peripheral vasculature. The vascular region may include one or more vessels, such as arteries, veins, capillaries, and so forth. By way of an example, the volumetric data 120 may represent a vascular region in the neural vasculature, and the neural vasculature may include an aneurysm and a feeding vessel that supplies blood to the aneurysm.

The volumetric data that is received in the operation S110 may be generated by various types of imaging systems. These include various volumetric imaging system such as computed tomography "CT" imaging systems, magnetic resonance imaging "MRI" systems, and 3D ultrasound imaging systems. The CT imaging system may be a spectral CT imaging system. In contrast to conventional CT imaging systems, and which generate X-ray attenuation data representing X-ray attenuation within a single energy interval, spectral CT imaging systems generate X-ray attenuation data representing X-ray attenuation within multiple different energy intervals. The X-ray attenuation data generated by a spectral CT imaging system may be processed, e.g. using various material decomposition algorithms, in order to distinguish between media that have similar X-ray attenuation values when measured within a single energy interval, and which would be indistinguishable in X-ray attenuation data obtained using a conventional CT imaging system. Thus, the X-ray attenuation data generated by spectral CT imaging systems may be processed to provide volumetric images with improved specificity to materials such as contrast agent, tissue, bone, and so forth.

In one example, the volumetric data that is received in the operation S110 is generated by a (spectral) projection X-ray imaging system. The (spectral) projection X-ray imaging system may be configured to acquire projection data representing the vascular region from multiple viewing angles with respect to the vascular region 130. A tomographic reconstruction of the projection data from the multiple viewing angles is then performed in order to provide volumetric data representing the vascular region 130. A volumetric image that is obtained using a (spectral) projection X-ray imaging system in this manner is sometimes referred-to as a 3D rotational angiogram "3DRA". By way of an example, volumetric data may be acquired in this manner by the projection X-ray imaging system 210 illustrated in Fig. 1. For instance, the projection X-ray imaging system 210, which includes an X-ray source 210^{S} and a corresponding X-ray detector 210^{D}, may be rotated around a vascular region in the brain whilst acquiring X-ray projection data representing the vascular region from multiple viewing angles with respect to the vascular region.

In some examples, the volumetric data 120 that is received in the operation S110 is generated subsequent to the injection of a contrast agent into the vasculature of a subject. In these examples, the volumetric data 120 represents a distribution of the contrast agent in the vascular region 130. A contrast agent serves to improve the distinction between blood and surrounding tissue in the volumetric data 120. In these examples, the volumetric data 120 may be referred-to as angiographic volumetric image data 120. Angiographic volumetric image data may be generated by a (spectral) CT imaging system, or by a (spectral) projection X-ray imaging system that acquires projection data from multiple viewing angles with respect to the vascular region. Angiographic volumetric image data may alternatively be generated by an MRI system.

The volumetric data 120 may be received from various sources in the operation S110. For example, the volumetric data 120 may be received from a medical imaging system such as one of the imaging systems described above. Alternatively, the volumetric data 120 may be received from another source, such as a computer readable storage medium, the Internet, the Cloud, and so forth. In general, the volumetric data 120 may be received by the processor(s) 110 via any form of data communication. The volumetric data 120 may be received via wired, or wireless, or optical fiber communication, for example. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 2; in this operation, the processor(s) determine, based on the volumetric data 120, and for each of a plurality of portions 130_{1..i} of the vascular region, a corresponding range of one or more viewing angles Δα_{1..i}, Δβ_{1..i} for acquiring X-ray projection data 150 representing the portion 130_{1..i}.

The portions 130_{1..i} of the vascular region for which the corresponding one or more viewing angles Δα_{1..i}, Δβ_{1..i} are determined, may in general be any portions of the vascular region 130. By way of an example, the vascular region may be a cardiovascular region and the portions 130_{1..i} of the vascular region may include segments of one or more coronary vessels within the cardiovascular region. By way of another example, the vascular region may be a neurovascular region, and the portions may correspond to an aneurysm, and a feeding vessel that supplies blood to the aneurysm, in a vascular region in the neural vasculature. This example is illustrated in Fig. 1, and wherein the aneurysm corresponds to the portion 130₁, and its feeding vessel corresponds to the portion 130₂. As described above, due to the lack of depth information in projection X-ray images, and due to the tortuous nature of the neural vasculature, it can be difficult to generate a projection X-ray image that simultaneously captures both the aneurysm and its feeding vessel, and for this image to be easy to interpret. By way of another example, the vascular region may be a neurovascular region, and the plurality of portions 130_{1..i} may correspond to different aneurysms. Here also, the distribution of the aneurysms confounds the capture of all aneurysms in a single image that is easy to interpret.

In general, there may be two or more portions 130_{1..i} of the vascular region. The portions may be defined in various ways, including manually, and automatically. In the former case, a user may define the portions in an image that is outputted by the processor(s) 120. For instance, in one example, the processor(s) 110 are configured to:
output an image representing the volumetric data 120;
receive user input defining the portions 130_{1..i} of the vascular region in the outputted image; and
determine the portions 130_{1..i} of the vascular region in the volumetric data 120 based on the received user input.

In this example, the outputted image may be generated by reconstructing the volumetric image. The outputted image may be a volumetric image, or an image slice, for example. The image may be outputted to a display device, such as the display 220 illustrated in Fig. 1, for example. The image may alternatively be outputted in a different manner, such as to a virtual/ augmented reality display device, or to a printer, or to a computer-readable storage medium, or to the Internet, or to the Cloud, and so forth. The user input may be received via a user input device, such as a touchscreen, or a pointing device (e.g. a mouse, or joystick), or a speech recognition device, or via another type of user input device. By way of an example, the user may provide this input via various colors, or rings, or contours (e.g. circles), or other ways of marking portions of vasculature.

In another example, the processor(s) 110 are configured to:
receive, from a projection X-ray imaging system, X-ray projection data representing the vascular region 130;
register the vascular region 130 represented by the X-ray projection data to the vascular region 130 represented by the volumetric data 120;
output an image representing the X-ray projection data;
receive user input defining the portions 130_{1..i} of the vascular region in the outputted image; and
determine the portions 130_{1..i} of the vascular region in the volumetric data 120 based on the received user input and the registration.

This example differs from the previous example in that the image in which the portions 130_{1..i} of the vascular region are defined, is generated from X-ray projection data representing the vascular region 130, rather than the volumetric data 120. The X-ray projection data may be generated by the projection X-ray imaging system 210 illustrated in Fig. 1. This data may be acquired in a pre-procedural phase, or in a peri-procedural phase. The registration between the vascular region 130 represented by the X-ray projection data, and the vascular region 130 represented by the volumetric data 120, may be performed using various known image registration techniques. This registration provides a mapping between the outputted image representing the X-ray projection data, and the vascular region 130 represented by the volumetric data 120, and is consequently used to define the portions 130_{1..i} of the vascular region in the volumetric data 120.

As mentioned above, the two or more portions 130_{1..i} may alternatively be defined automatically, instead of manually. In one example, the processor(s) 110 are configured to automatically determine the portions 130_{1..i} of the vascular region in the volumetric data 120. In this example, the portions may be defined automatically in the volumetric data 120, e.g. by using known image segmentation techniques to segment the vascular region, and thereby define the portions. In another example, the processor(s):
receive, from a projection X-ray imaging system, X-ray projection data representing the vascular region 130;
register the vascular region 130 represented by the X-ray projection data to the vascular region 130 represented by the volumetric data 120;
automatically determine the portions 130_{1..i} of the vascular region in the volumetric data 120 based on the projection X-ray data and the registration.

In this example, the X-ray projection data representing the vascular region 130 may be provided as described above for the manual definition of the portions, and the registration may likewise be performed using various known image registration techniques. The portions may be defined automatically in the projection X-ray data 120 by using known image segmentation techniques to segment the vascular region. The registration likewise provides a mapping between the projection X-ray data in which the portions are defined, and the volumetric data 120.

In one example, at least one of the portions 130_{1..i} is defined based on a detected position of an interventional device in the X-ray projection data. For instance, the position of an interventional device (e.g. a guidewire, catheter, treatment device, and so forth) may be detected in the projection X-ray data using known object detection techniques and used to define one of the portions 130_{1..i} based on its current position. The at least one of the portions 130_{1..i} may for example include a portion of the vascular region within which the interventional device is currently located, or a portion of the vascular region adjoining a distal end of the interventional device.

In the operation S120, various factors may be taken into account in determining the range of one or more viewing angles Δα_{1..i}, Δβ_{1..i} for each of the portions 130_{1..i} of the vascular region. For example, the processor(s) 110 may determine the ranges for the portions 130_{1..i} based on one or more of the following: a vessel overlap metric for the corresponding portion 130_{1..i}, a vessel foreshortening metric for the corresponding portion 130_{1..i}, a tissue thickness metric representing a tissue thickness presented to X-ray radiation passing through the corresponding portion 130_{1..i}, an image quality metric for the corresponding portion 130_{1..i}, an angular difference between the range for the corresponding portion 130_{1..i} and a range for another portion, a user-preferred range for the corresponding portion 130_{1..i}, a guideline-determined range for the corresponding portion 130_{1..i}.

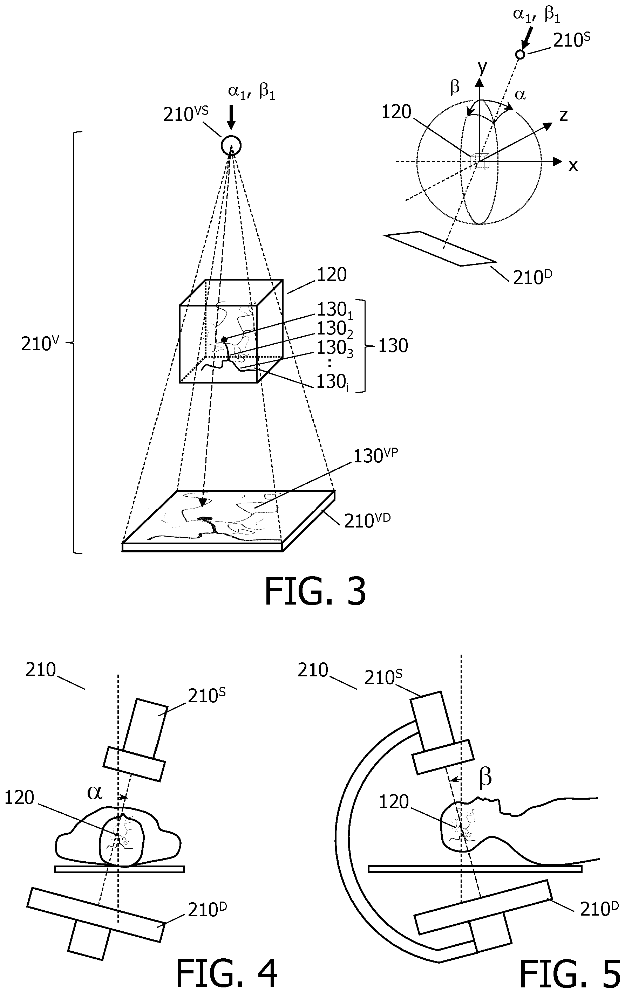

Some of the above-described factors may be determined based on virtual projections of the vascular region. For instance, in one example, the vessel overlap metric, the vessel foreshortening metric, the tissue thickness metric, and the image quality metric, are each determined based on virtual projections 130^{VP} of the corresponding portion 130_{1..i} from each of a plurality of candidate viewing angles α, β. The generation of these virtual projections from the volumetric data 120 that is received in the operation S120, is now described with reference to Fig. 3 - Fig. 5.

Fig. 3 is a schematic diagram illustrating an example of the generation of a virtual projection 130^{VP} of a vascular region 130 from a viewing angle α₁, β₁, of a virtual projection X-ray imaging system 210^{V} with respect to a vascular region 130, in accordance with some aspects of the present disclosure. The virtual projection X-ray imaging system 210^{V} illustrated in Fig. 3 includes virtual X-ray source 210^{VS} and a virtual X-ray detector 210^{VD}. The geometry of the virtual X-ray source 210^{VS} and the virtual X-ray detector 210^{VD} corresponds to the geometry of the X-ray source 210^{S} and the X-ray detector 210^{D} of the projection X-ray imaging system 210 illustrated in Fig. 1 that is subsequently used to acquire the projection data 150. For instance, the shape of the beam of X-ray radiation emitted by the virtual X-ray source 210^{VS} corresponds to that of the X-ray source 210^{S} illustrated in Fig. 1. Likewise, the areas of the X-ray detectors 210^{D} and 210^{VD} correspond to one another, and the relative positions of the X-ray sources 210^{S} and 210^{VS} and X-ray detectors 210^{D} and 210^{VD} in relation to the vascular region 130 correspond to one another. In the example illustrated in Fig. 3, the vascular region 130 is represented by the volumetric data 120. As described above, the volumetric data 120 may be generated by a volumetric imaging system, or it may be provided by projection X-ray imaging system that rotates around the vascular region 130 in order to acquire the volumetric data 120. The virtual projection X-ray imaging system 210^{V} illustrated in Fig. 3 has a viewing angle with respect to the vascular region 130. The viewing angle is defined by the angle α and the angle β with respect to the vascular region, as illustrated in the inset image in the upper right portion of Fig. 3. These angles are defined by the orientation of a central ray of the virtual X-ray source 210^{VS} with respect to the vascular region 130. In-turn, these angles are defined with respect to two anatomical axes of a subject, as described below. The viewing angle may alternatively be defined in a different manner. For instance, it may be defined by only one of the angles α, and β, or it may be defined in a different coordinate system, or with reference to different anatomical axes.

As mentioned above, the angle α illustrated in Fig. 3 is defined with respect to the vascular region 130, and this may in-turn be defined with respect to a subject's anatomical axes. In the example illustrated in Fig. 3, the angle α is defined with respect to the subject's ventral-dorsal axis, as shown in Fig. 4. Fig. 4 is a schematic diagram illustrating a first example of a viewing angle α of a projection X-ray imaging system 210 with respect to a vascular region 130, in accordance with some aspects of the present disclosure. In the example illustrated in Fig. 3, the angle β is defined with respect to the subject's cranial-caudal axis, as shown in Fig. 5. Fig. 5 is a schematic diagram illustrating a second example of a viewing angle β of a projection X-ray imaging system 210 with respect to a vascular region 130, in accordance with some aspects of the present disclosure.

The viewing angle of the virtual projection X-ray imaging system 210^{V} may alternatively be defined with respect to other anatomical axes. For instance, the viewing angle may also include a definition of a rotational angle of a central ray of the virtual X-ray source 210^{VS} with respect to the subject's anatomy.

Referring now to Fig. 3, the virtual X-ray source 210^{VS} and the virtual X-ray detector 210^{VD} may be used to generate a virtual projection 130^{VP} of a vascular region 130 from the viewing angle α, β, by projecting, in a mathematical sense, the volumetric data 120 representing the vascular region 120 onto the virtual X-ray detector 210^{VD} using virtual X-rays emitted from the virtual X-ray source 210^{VS}. For instance, at the viewing angle defined by α₁, and β₁, in Fig. 3, portions of the vascular region 130 lying along the path of the virtual X-ray indicated by the dashed arrowed line in Fig. 3 are projected onto the virtual detector 210^{VD} by calculating a line integral of the intensity values represented by the volumetric data 120 that lie along the path of the virtual X-ray to provide an image intensity value at a point in the virtual projection 130^{VP} at which the virtual X-ray intersects virtual X-ray detector 210^{VD}. The same operation may be repeated for multiple virtual X-rays that intersect different points across the virtual X-ray detector 210^{VD} to generate the virtual projection 130^{VP} of the vascular region 130.

As mentioned above, some of the above-described factors may be determined based on virtual projections of the vascular region. For instance, metrics such as a vessel overlap metric, a vessel foreshortening metric, a tissue thickness metric, and an image quality metric, may be calculated based on virtual projections of the vascular region. These factors may be calculated for the various portions 130_{1..i} of the vascular region, from the virtual projections 130^{VP} that are obtained from multiple candidate viewing angles α, β, as described in the examples below. In these examples, the candidate viewing angles for which the metric(s) are evaluated may be determined in various ways. For instance, the candidate viewing angles may be selected from an array of viewing angles defined by discrete positions of the virtual X-ray source 210^{VS} on a virtual sphere surrounding the vascular region 130. The complete set of viewing angles in the array may be selected, or a subset of the viewing angles in the array may be selected, based on considerations such as a feasibility criterion defining the achievability of the viewing angle taking into consideration factors such as a physician's access to the patient, a projection X-ray imaging system's ability to image a patient and so forth. The metric(s) may then be evaluated exhaustively for the viewing angles in the complete set/ subset. Alternatively, a search algorithm may be used wherein a subsequent candidate viewing angle for which the metric is to be evaluated, is determined based on the value of the metric at a current candidate viewing angle.

The vessel overlap metric quantifies the amount of overlap between the portion 130_{1..i}, and other vessels in the vascular region 130. The vessel overlap metric may be evaluated from a virtual projection 130^{VP} in various ways, for instance, a vessel overlap metric may be evaluated that represents the total number of vessels that overlap the portion, or the total area of vessel overlap regions that overlap the portion. The vessel overlap metric may be evaluated by casting virtual rays in the projection direction through the vascular region 130, and counting the number of virtual rays that intersect both the portion and also another vessel. Typically, it is desirable to minimize the vessel overlap metric. Thus, an optimal viewing angle that is determined based solely on the vessel overlap metric may be satisfied at a candidate viewing angle αₒₚₜ, βₒₚₜ at which these exists a minimum amount of vessel overlap for the portion; e.g. there exists a minimum total number of overlapping vessels, or a minimum total area of vessel overlap regions, for the portion.

The vessel foreshortening metric quantifies the amount of foreshortening of a portion 130_{1..i}. The vessel foreshortening metric may be evaluated from a virtual projection 130^{VP} in various ways. For instance, a vessel foreshortening metric, may be calculated that represents a (combined) length of one or more vessel segments in the portion in the virtual projection 130^{VP}. The (combined) length of the one or more vessel segments can be calculated explicitly, e.g. based on the length(s) of projections of the vessel segment(s). Alternatively a surrogate measure of the combined length may be calculated. For example, a surrogate measure of the combined length may be calculated by evaluating the inner products of the tangential centerline vectors of one or more vessel segments in the vascular region with their corresponding projection directions. Typically, it is desirable to minimize the vessel foreshortening metric, and so an optimal viewing angle that is determined based solely on the vessel foreshortening metric may be satisfied at a candidate viewing angle αₒₚₜ, βₒₚₜ at which there is minimum vessel foreshortening; e.g. there exists a maximum (combined) length of the one or more vessel segments in the portion.

The tissue thickness metric quantifies the thickness of tissue along the projection direction through the portion. The tissue thickness metric may be evaluated from a virtual projection 130^{VP} in various ways. For instance, the Hounsfield Units "HU" values that are within a range that corresponds to tissue, may be integrated along virtual rays along the projection direction through the portion. The integrated HU values may be summed for the portion in order to provide a metric representing the tissue thickness metric for the portion. Typically, it is desirable to minimize the tissue thickness metric, and so an optimal viewing angle that is determined based solely on the tissue thickness metric may be satisfied at a candidate viewing angle αₒₚₜ, βₒₚₜ at which the tissue thickness metric has a minimum value.

The image quality metric quantifies the image quality of the portion. The image quality metric may be evaluated from a virtual projection 130^{VP} based on one or more image quality components. For instance, image quality components such as an amount of noise, or a sharpness/ blur, image contrast, may be evaluated for the portion in the virtual projection 130^{VP}. Other examples of image quality components include measures of image artifacts such as blooming artifacts, or streaks due to metal artifacts and so forth. Typically, it is desirable to maximize the image quality metric, and so an optimal viewing angle that is determined based solely on the image quality metric may be satisfied at a candidate viewing angle αₒₚₜ, βₒₚₜ at which the image quality metric has a maximum value.

As mentioned above, other factors that may be taken into account in determining the range of one or more viewing angles Δα_{1..i}, Δβ_{1..i} for each of the portions 130_{1..i} of the vascular region in the operation S120 include an angular difference between the range for the corresponding portion 130_{1..i} and a range for another portion, or a user-preferred range for the corresponding portion 130_{1..i}, or a guideline-determined range for the corresponding portion 130_{1..i}. It may be desirable to minimize the angular difference between the range for the corresponding portion 130_{1..i} and a range for another portion, e.g. an adjacent portion, or a contiguous portion, because a large angular difference can result in second order, and also higher order, jumps in the path of a vessel in the images that are generated from the acquired X-ray projection data. A user-preferred range may be retrieved from a database that stores the preferred range for various users. A guideline-determined range may be derived from automated angle suggestions as per existing clinical guidelines as for vasculature imaging, and which provide that the portion, or the portions, within the vascular region, are imaged, e.g. using complementary, or orthogonal views.

As mentioned above, one or more of the above-described factors may thus be taken into account in determining the range of one or more viewing angles Δα_{1..i}, Δβ_{1..i} for acquiring X-ray projection data 150 representing each portion 130_{1..i}. In one example, the processor(s) 110 are configured to determine the ranges Δα_{1..i}, Δβ_{1..i} for the portions 130_{1..i} based on a value of a cost function. The cost function is evaluated for the corresponding portion 130_{1..i} by applying weights to the one or more factors.

In this example, the weights are therefore used to determine the relative importance of the corresponding factors. The values of the weights may be set in various ways. For instance, in one example, the values of the weights are set empirically. In another example, the values of the weights are user-adjustable. A user may provide the values of the weights via a user interface device, for example. In another example, the values of the weights are updated automatically if the user uses different viewing angles to acquire projection data to the viewing angles that are outputted by the processor(s). In this example, the processor(s) 110 are configured to:
acquire, from a projection X-ray imaging system, actual viewing angle data representing actual viewing angles used to acquire X-ray projection data representing the portions 130_{1..i} of the vascular region; and
update a value of one or more of the weights based on an angular difference between the actual viewing angles and the viewing angles Δα_{1..i}, Δβ_{1..i}, Δαₒₚₜ, Δβₒₚₜ determined by the one or more processors.

Thus, in this example, the system 100 learns to adapt the weights so as to conform to the preferences of a user. For example, if a user values minimal foreshortening over minimal vessel overlap, as determined from the actual viewing angle data, the system adapts to such preferences.

In general, in the operation S120 , there may be a single, i.e. one and only one, viewing angle that is determined per portion 130_{1..i}, or alternatively, a range of viewing angles may be determined per portion 130_{1..i}. A single viewing angle may be determined per portion by selecting the value of the viewing angle for which the value of the cost function is optimal, e.g. maximized, or minimized, as described above. A range of viewing angles may be determined per portion by selecting the range of viewing angles for which the value of the cost function meets a predetermined criterion, e.g. the value of the cost function is greater than or equal to a threshold value, or the value of the cost function is less than or equal to a threshold value.

Referring now to the operation S130 illustrated in Fig. 2; in this operation, the processor(s) 110 are configured to output the ranges for the portions 130_{1..i} and/or an optimal range of one or more viewing angles Δαₒₚₜ, Δβₒₚₜ for acquiring X-ray projection data 150 representing the plurality of portions 130_{1..i}, the optimal range being determined based on the ranges for the portions 130_{1..i}.

In the first of these options, the ranges may be outputted to a display device, such as the display 220 illustrated in Fig. 1, for example. The ranges may be outputted in the form of a table, or as an image indicating the portions with corresponding text indicating the range for each portion. The ranges may alternatively be outputted in a different manner. The ranges may alternatively be outputted to a different display device such as a virtual/ augmented reality display device; or they may be outputted to a printer, or to a computer-readable storage medium, or to the Internet, or to the Cloud, and so forth. By outputting, for each portion, the range of one or more viewing angles Δα_{1..i}, Δβ_{1..i} for acquiring X-ray projection data 150 representing the portion 130_{1..i}, an operator may determine how to acquire X-ray projection data 150 that provides a view of the vasculature that is easy to interpret.

In the second of these options, the optimal range may be outputted in a similar manner to that described above for the ranges. However, in contrast to the ranges for the individual portions, the optimal range includes one or more viewing angles Δαₒₚₜ, Δβₒₚₜ for acquiring X-ray projection data 150 representing a plurality of the portions 130_{1..i}, or in other words, a combination of the portions. The optimal range is determined based on the ranges for the individual portions 130_{1..i}.

The optimal range Δαₒₚₜ, Δβₒₚₜ for acquiring X-ray projection data 150 representing the plurality of portions 130_{1..i} may be calculated in various ways from the ranges Δα_{1..i}, Δβ_{1..i} for the individual portions. In general, the optimal range Δαₒₚₜ, Δβₒₚₜ may correspond to a single viewing angle, i.e. one and only one viewing angle. In other examples, the optimal range includes multiple viewing angles.

In some examples, the optimal range is defined by an overlapping portion of two or more of the ranges for the individual portions 130_{1..i}. By way of an example, if there are two portions, and the ranges for these portions overlap one another, then the overlapping portion of these ranges may be deemed to be the optimal range. In some examples, the ranges for the individual portions are determined in terms of only one coordinate, e.g. Δα_{1..i}. In such situations it is sufficient that the ranges overlap for this coordinate alone. In other examples, the ranges for the individual portions are determined in terms of two or more coordinates, e.g. Δα_{1..i} and Δβ_{1..i}. In such situations, the ranges are deemed to overlap only over the extent of α and β for which both ranges overlap one another. If there are more than two ranges and the ranges do not all share a common overlapping range, then the portions may be grouped based on the portions that do have overlapping ranges. Thus, an optimal range may be determined for each of multiple groups of two of more portions for which an overlapping range exists.

In a related example, the optimal range is determined by an overlapping portion of two or more of the ranges for the portions 130_{1..i} within which the values of the cost function for the corresponding portions 130_{1..i} meet a predetermined criterion. In this example, the predetermined criterion may for instance specify that the value of the cost function is greater than or equal to a threshold value, or the value of the cost function is less than or equal to a threshold value. The threshold value may be adjusted in order to provide a wide, or a narrow optimal range. In one example, the predetermined criterion may specify that the value of the cost function is maximized, or minimized. This may be used to specify a range that corresponds to a single viewing angle, i.e. one and only one viewing angle, for acquiring X-ray projection data 150 representing the plurality of portions 130_{1..i}.

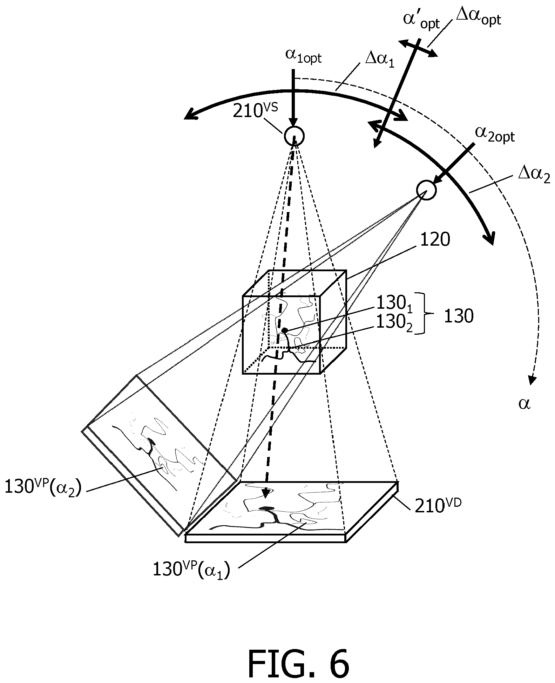

Examples of the above-described ranges are illustrated in Fig. 6, which is a schematic diagram illustrating examples of ranges of viewing angles Δα₁ and Δα₂ for acquiring X-ray projection data representing individual portions 130₁ and 130₂ of a vascular region, and also an optimal range of viewing angles Δαₒₚₜ for acquiring X-ray projection data representing both portions 130₁, and 130₂, in accordance with some aspects of the present disclosure. In the example illustrated in Fig. 6, the portion 130₁ corresponds to an aneurysm, and the portion 130₂ corresponds to its feeding vessel. Virtual projections 140_{VP}(α₁), and 140_{VP}(α₂), have been generated from the candidate viewing angles α₁, and α₁, respectively, and these include the portions 130₁ and 130₂. The value of a cost function has been evaluated for the portions 130₁ and 130₂ for each of multiple candidate viewing angles by applying the various weighted factor(s) described above to the corresponding virtual projections. For the portion 130₁, the value of the cost function has been deemed to meet a predetermined criterion (e.g. there is an acceptable amount of both foreshortening and overlap) for the range of viewing angles Δα₁. Thus, the range of viewing angles Δα₁ has been deemed to be acceptable for acquiring projection X-ray data of the portion 130₁. An optimal viewing angle has been found for the portion 130₁ at the angle α₁ₒₚₜ (e.g. there is a minimum amount of both foreshortening and overlap at this angle). A similar analysis has been performed for the portion 130₂, and the range of viewing angles Δα₂ has been deemed to be acceptable for acquiring projection X-ray data of the portion 130₂. An optimal viewing angle has been found for the portion 130₂ at the angle α₂ₒₚₜ (e.g. there is a minimum amount of both foreshortening and overlap at this angle). Thus, in this example, the ranges of viewing angles Δα₁ and Δα₂ may be outputted for the portions 130₁ and 130₂. Instead of outputting the ranges of multiple viewing angles Δα₁ and Δα₂*,* the optimal viewing angles may instead be outputted for the portions 130₁ and 130₂; i.e. the optimal angle α₁ₒₚₜ may be outputted for the portion 130₁, and the optimal angle α₂ₒₚₜ may be outputted for the portion 130₂.

In the example illustrated in Fig. 6, the ranges of viewing angles Δα₁ and Δα₂ for the portions 130₁ and 130₁, can be seen to overlap one another. In this case, there is an optimal range of viewing angles Δαₒₚₜ for acquiring X-ray projection data representing both of the portions 130₁ and 130₂. Thus, instead of (or in addition to) outputting the ranges of viewing angles Δα₁ and Δα₂ described above for the individual portions 130₁ and 130₂, the optimal range of viewing angles Δαₒₚₜ for acquiring X-ray projection data representing both of the portions 130₁ and 130₂, may instead be outputted.

If, however, none of the ranges for the individual portions overlap, or if none of these ranges overlap whilst simultaneously satisfying the predetermined criteria for the individual portions, then the ranges for the individual portions may be outputted, as described in the option above.

One or more additional operations may be performed by the processor(s) 110 of the system described above with reference to Fig. 1, as described in the examples below.

In one example, the outputting that is performed in the operation S130 described above, is performed based on a deviation between a current viewing angle of the projection X-ray imaging system with respect to the anatomical region, and the viewing angle that is determined by the system 100 in the operation S120. In this example, the processor(s) 110 are configured to:
receive, from a projection X-ray imaging system, X-ray projection data representing the vascular region 130;
determine a current viewing angle of the projection X-ray imaging system with respect to the anatomical region based on a registration between the X-ray projection data and the volumetric data 120; and
wherein the one or more processors 110 are configured to perform the outputting in response to: an angular difference between a current viewing angle for one of the portions 130_{1..i} and the range of one or more viewing angles Δα_{1..i}, Δβ_{1..i} for the portion 130_{1..i}, exceeding a predetermined value.

In this example, the outputting of the range of one or more viewing angles Δα_{1..i}, Δβ_{1..i} for the portion 130_{1..i} is therefore performed if the current viewing angle for a portion deviates by too much. Thus, the system guides a radiologist to use improved viewing angles for the portions. In this example, the X-ray projection data representing the vascular region 130 is generated by the same projection X-ray imaging system that is used to acquire the X-ray projection data 150. Thus, the X-ray projection data may be acquired by the projection X-ray imaging system 210 illustrated in Fig. 1. The X-ray projection data may be acquired during an initial positioning scan, for example. The registration between the X-ray projection data and the volumetric data 120 may be performed using known image registration techniques.

In a related example, instead of performing the outputting in response to an angular difference between a current viewing angle for one of the portions 130_{1..i} and the range of one or more viewing angles Δα_{1..i}, Δβ_{1..i} for the portion 130_{1..i}, exceeding a predetermined value, the processor(s) 110 are configured to perform the outputting in response to:
an angular difference between a current viewing angle for the plurality of portions 130_{1..i} and the optimal range for the plurality of portions 130_{1..i}, exceeding a predetermined value.

Thus, as in the example above, the system similarly guides a radiologist to use improved viewing angles, in this case for the optimal range for the plurality of portions 130_{1..i}.

In another example, the operation of outputting the ranges for the portions 130_{1..i} comprises outputting a suggested value of a viewing angle within the ranges. In this example, the processor(s) 110 are configured to:
receive user input confirming the suggested value of a viewing angle; and
automatically adjust a viewing angle of the projection X-ray imaging system to provide the suggested value of the viewing angle.

In this example, the suggested value may be the optimal value within a range that is determined for a portion. Thus, in this example, the value α₁ₒₚₜ may be suggested for the range Δα₁, and the value α₂ₒₚₜ may be suggested for the range Δα₂*.* The automatic adjustment of the viewing angle reduces delays in acquiring the X-ray projection data 150.

In another example, the operation of outputting an optimal range comprises outputting a suggested value of a viewing angle within the optimal range. In this example, the processor(s) 110 are configured to:
receive user input confirming the suggested value of a viewing angle; and
automatically adjust a viewing angle of the projection X-ray imaging system to provide the suggested value of the viewing angle.

In this example, the value α'ₒₚₜ may be suggested for the range Δαₒₚₜ. The automatic adjustment of the viewing angle that is provided by this example likewise reduces delays in acquiring the X-ray projection data 150.

In another example, the processor(s) 110 are configured to determine, and output, a value of at least one of the following:
a value of a collimation parameter for acquiring the X-ray projection data 150 from the outputted one or more viewing angles Δα_{1..i}, Δβ_{1..i}, Δαₒₚₜ, Δβₒₚ;
a value of an X-ray dose for acquiring the X-ray projection data 150 from the outputted one or more viewing angles Δα_{1..i}, Δβ_{1..i}, Δαₒₚₜ, Δβₒₚ.

This example provides proposed image acquisition parameters for the projection X-ray imaging system that will acquire the X-ray projection data 150. The proposed parameters facilitate the X-ray projection data 150 to be acquired in a consistent, and also efficient manner. The value of the collimation parameter, and likewise the value of the X-ray dose, may be determined in various ways, such as from a lookup table, for example. The value of the collimation parameter, and likewise the value of the X-ray dose, may be outputted to a display device, such as the display 220 illustrated in Fig. 1.

In a related example, the processors are also configured to receive user input indicating a confirmation of the outputted value(s), and the value is automatically applied to the projection X-ray imaging system. This reduces delays in acquiring the X-ray projection data 150. In another related example, processor(s) are configured to adjust the position of a subject bed (illustrated in Fig. 1) in order to position the vascular region optimally with respect to the projection X-ray imaging system.

It is noted that in addition to the processor(s) 110, the system 100 may also include one or more of: a projection X-ray imaging system for acquiring the X-ray projection data 150, such as for example the projection X-ray imaging system 210 illustrated in Fig. 1; a display device, such as the display 220 illustrated in Fig. 1, for displaying the ranges, other outputs generated by the processor(s) 110, and so forth; an injector (not illustrated in Fig. 1) for injecting a contrast agent into the subject; a subject bed 230; and a user input device (not illustrated in Fig. 1) configured to receive user input in relation to the operations performed by the processor(s) 110, such as a keyboard, a mouse, a touchscreen, speech recognition device, and so forth.

In another example, a computer-implemented method of providing viewing angles for acquiring X-ray projection data representing a vascular region, is provided. The method includes:
receiving S110 volumetric data 120 representing the vascular region 130;
determining S120, based on the volumetric data 120, and for each of a plurality of portions 130_{1..i} of the vascular region, a corresponding range of one or more viewing angles Δα_{1..i}, Δβ_{1..i} for acquiring X-ray projection data 150 representing the portion 130_{1..i}; and
outputting S130 the ranges for the portions 130_{1..i} and/or an optimal range of one or more viewing angles Δαₒₚₜ, Δβₒₚₜ for acquiring X-ray projection data 150 representing the plurality of portions 130_{1..i}, the optimal range being determined based on the ranges for the portions 130_{1..i}.

In another example, a computer program product, is provided. The computer program product comprises instructions which when executed by one or more processors, cause the one or more processors to carry out a method of providing viewing angles for acquiring X-ray projection data representing a vascular region, is provided. The method includes:
receiving S110 volumetric data 120 representing the vascular region 130;
determining S120, based on the volumetric data 120, and for each of a plurality of portions 130_{1..i} of the vascular region, a corresponding range of one or more viewing angles Δα_{1..i}, Δβ_{1..i} for acquiring X-ray projection data 150 representing the portion 130_{1..i}; and
outputting S130 the ranges for the portions 130_{1..i} and/or an optimal range of one or more viewing angles Δαₒₚₜ, Δβₒₚₜ for acquiring X-ray projection data 150 representing the plurality of portions 130_{1..i}, the optimal range being determined based on the ranges for the portions 130_{1..i}.

The above examples are to be understood as illustrative of the present disclosure, and not restrictive. Further examples are also contemplated. For instance, the examples described in relation to a system 100, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing viewing angles for acquiring X-ray projection data representing a vascular region, the system comprising one or more processors (110) configured to:
receive (S110) volumetric data (120) representing the vascular region (130);
determine (S120), based on the volumetric data (120), and for each of a plurality of portions (130_{1..i}) of the vascular region, a corresponding range of one or more viewing angles (Δα_{1..i}, Δβ_{1..i}) for acquiring X-ray projection data (150) representing the portion (130_{1..i}); and
output (S130) the ranges for the portions (130_{1..i}) and/or an optimal range of one or more viewing angles (Δαₒₚₜ, Δβₒₚₜ) for acquiring X-ray projection data (150) representing the plurality of portions (130_{1..i}), the optimal range being determined based on the ranges for the portions (130_{1..i}).

2. The system according to claim 1, wherein the one or more processors (110) are configured to determine the ranges for the portions (130_{1..i}) based on one or more of the following factors for the corresponding portion (130_{1..i}): a vessel overlap metric, a vessel foreshortening metric, a tissue thickness metric representing a tissue thickness presented to X-ray radiation passing through the portion (130_{1..i}), an image quality metric, an angular difference between the range and a range for another portion, a user-preferred range, a guideline-determined range.

3. The system according to claim 2, wherein the one or more processors (110) are configured to determine the vessel overlap metric and/or the vessel foreshortening metric and/or the tissue thickness metric and/or the image quality metric, based on virtual projections (130^{VP}) of the corresponding portion (130_{1..i}) from each of a plurality of candidate viewing angles (α, β).

4. The system according to claim 2 or claim 3, wherein the one or more processors (110) are configured to determine the ranges (Δα_{1..i}, Δβ_{1..i}) for the portions (130_{1..i}) based on a value of a cost function, and wherein the cost function is evaluated for the corresponding portion (130_{1..i}) by applying weights to the one or more factors.

5. The system according to claim 4, wherein the one or more processors (110) are further configured to:
acquire, from a projection X-ray imaging system, actual viewing angle data representing actual viewing angles used to acquire X-ray projection data representing the portions (130_{1..i}) of the vascular region; and
update a value of one or more of the weights based on an angular difference between the actual viewing angles and the viewing angles (Δα_{1..i}, Δβ_{1..i}, Δαₒₚₜ, Δβₒₚₜ) determined by the one or more processors.

6. The system according to claim 4, wherein at least one of the portions 130_{1..i} is defined based on a detected position of an interventional device in the X-ray projection data.

7. The system according to any previous claim, wherein the optimal range is defined by an overlapping portion of two or more of the ranges for the portions (130_{1..i}).

8. The system according to claim 7 when dependent on claim 4, wherein the optimal range is determined by an overlapping portion of two or more of the ranges for the portions (130_{1..i} ) within which the values of the cost function for the corresponding portions (130_{1..i}) meet a predetermined criterion.

9. The system according to any previous claim, wherein the one or more processors (110) are further configured to:
output an image representing the volumetric data (120);
receive user input defining the portions (130_{1..i}) of the vascular region in the outputted image; and
determine the portions (130_{1..i}) of the vascular region in the volumetric data (120) based on the received user input.
or
receive, from a projection X-ray imaging system, X-ray projection data representing the vascular region (130);
register the vascular region (130) represented by the X-ray projection data to the vascular region (130) represented by the volumetric data (120);
output an image representing the X-ray projection data;
receive user input defining the portions (130_{1..i}) of the vascular region in the outputted image; and
determine the portions (130_{1..i}) of the vascular region in the volumetric data (120) based on the received user input and the registration.

10. The system according to any one of claims 1 - 8, wherein the one or more processors (110) are further configured to:
automatically determine the portions (130_{1..i}) of the vascular region in the volumetric data (120);
or
receive, from a projection X-ray imaging system, X-ray projection data representing the vascular region (130);
register the vascular region (130) represented by the X-ray projection data to the vascular region (130) represented by the volumetric data (120);
automatically determine the portions (130_{1..i}) of the vascular region in the volumetric data (120) based on the projection X-ray data and the registration.

11. The system according to any previous claim, wherein the vascular region is a neurovascular region; and
wherein the plurality of portions (130_{1..i}) of the vascular region comprise different aneurysms;
or
wherein the plurality of portions (130_{1..i}) of the vascular region comprise an aneurysm, and a feeding vessel of the aneurysm.

12. The system according to any one of claims 1 - 10, wherein the vascular region is a cardiovascular region; and
wherein the plurality of portions (130_{1..i}) of the vascular region comprise segments of one or more coronary vessels within the cardiovascular region.

13. The system according to any previous claim, wherein the one or more processors (110) are further configured to:
receive, from a projection X-ray imaging system, X-ray projection data representing the vascular region (130);
determine a current viewing angle of the projection X-ray imaging system with respect to the anatomical region based on a registration between the X-ray projection data and the volumetric data (120); and
wherein the one or more processors (110) are configured to perform the outputting in response to:
an angular difference between a current viewing angle for one of the portions (130_{1..i}) and the range of one or more viewing angles (Δα_{1..i}, Δβ_{1..i}) for the portion (130_{1..i}), exceeding a predetermined value;
or
an angular difference between a current viewing angle for the plurality of portions (130_{1..i}) and the optimal range for the plurality of portions (130_{1..i}), exceeding a predetermined value.

14. The system according to claim 13, wherein the outputting the ranges for the portions (130_{1..i}) comprises outputting a suggested value of a viewing angle within the ranges and/or wherein the outputting an optimal range comprises outputting a suggested value of a viewing angle within the optimal range; and
wherein the one or more processors (110) are further configured to:
receive user input confirming the suggested value of a viewing angle; and
automatically adjust a viewing angle of the projection X-ray imaging system to provide the suggested value of the viewing angle.

15. The system according to any previous claim, wherein the one or more processors (110) are further configured to determine, and to output, a value of at least one of the following:
a value of a collimation parameter for acquiring the X-ray projection data (150) from the outputted one or more viewing angles (Δα_{1..i}, Δβ_{1..i}, Δαₒₚₜ, Δβₒₚ);
a value of an X-ray dose for acquiring the X-ray projection data (150) from the outputted one or more viewing angles (Δα_{1..i}, Δβ_{1..i}, Δαₒₚₜ, Δβₒₚ).
